# EUROPEAN PATENT SPECIFICATION

(11) **EP 2 019 319 B1**
(45) Date of publication and mention of the grant of the patent: **19.10.2016**
(21) Application number: 08161051.1
(22) Date of filing: 24.07.2008
(51) Int. Cl.: G01N 33/543, G01N 33/531, C08F 220/56

(54) **Reagent for latex aggregation reaction and method for detecting target substance**
Reagenz für eine Latexaggregationsreaktion und Verfahren zum Nachweis einer Zielsubstanz
Réactif pour réaction d'agrégation de latex et procédé de détection de substance cible

(30) Priority: 25.07.2007 JP 2007193688
(43) Date of publication of application: 28.01.2009
(73) Proprietor: JSR Corporation, Minato-ku Tokyo 105-7640 (JP)
(72) Inventor: Fan, Kejun, Chuo-ku Tokyo 104-8410 (JP); Tamori, Kouji, Chuo-ku Tokyo 104-8410 (JP)
(74) Representative: TBK

(56) References cited:
- EP-A1- 1 061 368
- JP-A- 8 278 308
- US-A- 4 128 520
- RENIL M ET AL.: "Synthesis and application of a PEGA polymeric support for high capacity continuous flow solid-phase peptide synthesis" TETRAHEDRON LETTERS, vol. 36, no. 26, 26 June 1995 (1995-06-26) , pages 4647-4650, XP4027835 GB ISSN: 0040-4039 DOI: 10.1016/0040-4039(95)00808-P

## Description

### BACKGROUND OF THE INVENTION

### 1. Field the Invention

The present invention relates to a reagent for latex aggregation, its use and a method for detecting a target substance.

### 2. Brief Description of the Background Art

In order to measure a specific antigen or antibody (target substance) utilizing aggregation reaction through antigen-antibody, a method for measuring immunolatex aggregation wherein an immunoreactive substance such as an antibody or an antigen is supported on carrier particles and a target substance such as a corresponding antigen or antibody is detected through a specific reaction has been widely used in the regions of clinical laboratory tests, biochemical studies, and the like, (e.g., JP-A-2006-266970).

In the method for measuring immunolatex aggregation, generally, carrier particles on which an antibody (antigen) against a target antigen (antibody) to be measured is immobilized are brought into contact with an analyte which possibly contains the target substance and, in the case where the target substance is present, aggregation reaction of the particles, which occurs in the mixed liquid as a result of an antigen-antibody reaction, is detected.

However, in the method for measuring immunolatex aggregation, there is a measurement limit in the detection of light absorption and insufficient detection sensitivity to particle aggregation is pointed out in comparison with the other measuring methods (e.g., an enzyme-multiplication method, a chemiluminescence measuring method).

In the method for measuring immunolatex aggregation, in order to carrying out more highly sensitive measurement, usually, carrier particles having larger particle diameter are used. However, from the limitation of measuring wavelength, when the particle diameter of the carrier particles exceeds a certain upper limit, a measurable range remarkably decreases and also the precipitating property of the carrier particles increases to such a degree which cannot be ignored within an actual measuring time. Therefore, it is difficult to markedly enlarge the particle diameter of carrier particles.

On the other hand, by using carrier particles having a small particle diameter, a large number of studies have been performed for obtaining a large measurement sensitivity. For example, in JP-A-8-278308, JP-A-2003-294753, JP-A-2006-105910, and the like, in order to promote immunoreaction of carrier particles, a water-soluble polymer having an aggregation-promoting function for the carrier particles under certain conditions is disclosed. Examples of such a water-soluble polymer include polyethylene glycol, polyhydric alcohols, polyvinylpyrrolidone, carboxymethyl cellulose, saccharose, dextran, polyamino acids, alginic acid, aminoethanesulfonic acid derivatives, aminopropanesulfonic acid derivatives, and the like.

However, any of these water-soluble polymers exhibit a restrictive sensitizing effect in the particle aggregation reaction and, in most cases, they have fatal defect that they cause aggregation of particles by a factor other than the antigen-antibody reaction. Therefore, the applications and use conditions of the aforementioned water-soluble polymers are limited. Furthermore, since solution of any of the aforementioned water-soluble polymers has high solution viscosity even in the case where the concentration is low, carrier particles are heterogeneously present in an immunoreaction system. Therefore, there are cases where measured results vary or measurement accuracy is low.

EP 1 061 368 A1 is directed to an agglutination assay method in a binder medium, which is a so-called *"dry analysis",* wherein the agglutination is not carried out in a system where all components are fully dissolved in a medium but wherein a dry analysis element is applied.

### SUMMARY OF THE INVENTION

The present invention provides use of a reagent for latex aggregation reaction capable of promoting immunoreaction of particles wherein an antibody or antigen is immobilized with an antigen or antibody to accelerate aggregation of the particles; and suppressing increase in background and occurrence of non-specific reaction, and a method for detecting a target substance.

### DETAILED DESCRIPTION OF THE INVENTION

The reagent for latex aggregation reaction used in the present invention comprises a water-soluble polymer having a repeating unit represented by the following formula (1): 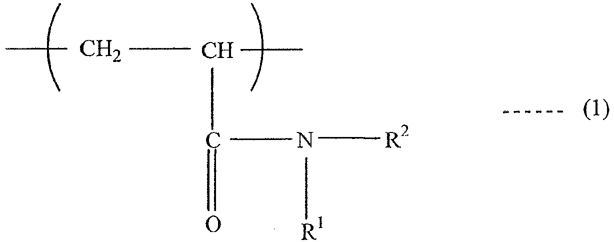 wherein R¹ and R² each independently represents a hydrogen atom or a substituted or unsubstituted alkyl group having 1 to 8 carbon atoms.

In the present invention, the "water-soluble polymer" means a polymer which provides a visually clear solution when the polymer is added to and mixed with pure water in order to adjust a solid content to be 1% at 25°C.

In the above reagent for latex aggregation reaction, the above water-soluble polymer can further have a repeating unit represented by the following formula (2): 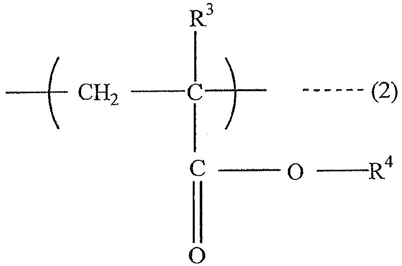 wherein R³ represents a hydrogen atom or a methyl group and R⁴ represents a substituted or unsubstituted alkyl group having 1 to 12 carbon atoms, an alicyclic hydrocarbon group, or an aromatic hydrocarbon group.

In the reagent for latex aggregation reaction, the above water-soluble polymer may be at least one selected from polyacrylamide, polydimethylacrylamide, and a copolymer of an acrylamide monomer with a (meth)acrylate monomer.

The method for detecting a target substance according to one embodiment of the present invention is defined in claim 1.

In the above method for detecting a target substance, the above water-soluble polymer as defined in claim 1 can further have a repeating unit represented by the following formula (2): 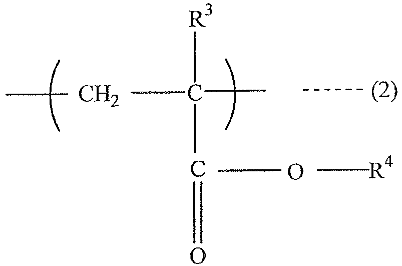 wherein R³ represents a hydrogen atom or a methyl group and R⁴ represents a substituted or unsubstituted alkyl group having 1 to 12 carbon atoms, an alicyclic hydrocarbon group, or an aromatic hydrocarbon group.

In the above method for detecting a target substance, the above water-soluble polymer as defined in claim 1 may be at least one selected from polyacrylamide, polydimethylacrylamide, and a copolymer of an acrylamide-based monomer with a (meth)acrylate monomer.

In a method for measuring immunolatex aggregation, by detecting a target substance using a reagent for latex aggregation reaction containing a water-soluble polymer represented by the above formula (1), the aggregation reaction of particles can be amplified to enhance sensitivity for detecting the target substance. Furthermore, in comparison with the case where the other water-soluble polymer is used as a sensitizer, occurrence of non-specific reaction can be suppressed. As a result, reproducibility and accuracy of the measurement of the latex aggregation reaction can be improved.

Moreover, the above reagent for latex aggregation reaction is easy to handle and deterioration with lapse of time is very little, so that storage stability is satisfactory.

Furthermore, according to the above method for detecting a target substance, a wide measurable range, and a high measurement sensitivity and quantitative determination ability can be achieved by including a step of mixing a water-soluble polymer having a repeating unit represented by the above formula (1); particles to which an antibody or antigen for a predetermined target substance is immobilized; and an analyte, and a step of optically detecting aggregation of the particles formed in the mixing step.

The following will specifically describe a reagent for latex aggregation reaction and a method for detecting a target substance according to one embodiment of the present invention.

### 1. Reagent for latex aggregation reaction

The reagent for latex aggregation reaction for use in the present invention contains a water-soluble polymer having a repeating unit represented by the following formula (1): 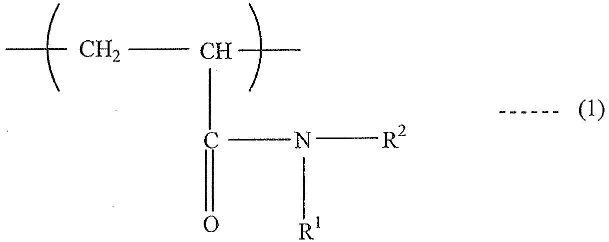 wherein R¹ and R² each independently represents a hydrogen atom or a substituted or unsubstituted alkyl group having 1 to 8 carbon atoms.

### 1.1. Water-soluble polymer

In the reagent for latex aggregation reaction according to the present embodiment, the water-soluble polymer has a function as a sensitizer.

In the above formula (1), examples of the substituted or unsubstituted alkyl group having 1 to 8 carbon atoms represented by R¹ and R² include a methyl group, an ethyl group, an n-propyl group, an isopropyl group, an n-butyl group, a sec-butyl group, a tert-butyl group, a pentyl group, a hexyl group, a heptyl group, an octyl group, and groups wherein these groups are substituted by a functional group such as a hydroxy group, or an alkoxy group. As R¹ and R², a hydrogen atom or a methyl group is more preferable.

Moreover, the water-soluble polymer may further have a repeating unit represented by the following formula (2): 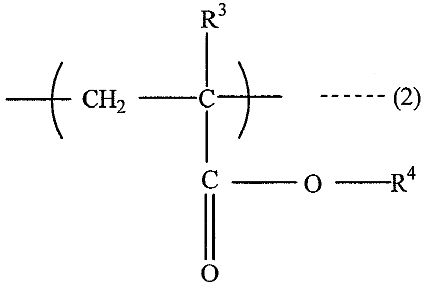 wherein R³ represents a hydrogen atom or a methyl group and R⁴ represents a substituted or unsubstituted alkyl group having 1 to 12 carbon atoms, an alicyclic hydrocarbon group, or an aromatic hydrocarbon group.

In the above formula (2), examples of the substituted or unsubstituted alkyl group having 1 to 12 carbon atoms represented by R⁴ includes R¹ and R². As R⁴, at least one selected from a methyl group, an ethyl group, and a methoxyethyl group is more preferable and a methoxyethyl group is further preferable.

Moreover, in the above formula (2), examples of the alicyclic hydrocarbon group represented by R⁴ include an isobornyl group and an cyclohexyl group. Examples of the aromatic hydrocarbon group represented by R⁴ include a benzyl group.

The water-soluble polymer may be a homopolymer consisting of a repeating unit represented by the above formula (1) alone, such as polyacrylamide or polydimethylacrylamide or may be a heteropolymer (copolymer) containing a repeating unit represented by the above formula (1) and the other repeating unit(s).

For example, it is preferable that the water-soluble polymer is at least one selected from polyacrylamide, polydimethylacrylamide, and a copolymer of an acrylamide monomer with a (meth)acrylate monomer. Examples of the copolymer of an acrylamide monomer with a (meth)acrylate monomer include a dimethylacrylamide-methoxyethyl acrylate copolymer.

In the case where the dimethylacrylamide-methoxyethyl acrylate copolymer is used as the water-soluble polymer, it is preferable that the ratio of the repeating unit represented by the above formula (1) (dimethylacrylamide: R¹ and R² are both a methyl group) to the repeating unit represented by the above formula (2) (methoxyethyl acrylate: R³ is a hydrogen atom and R⁴ is a methoxyethyl group) is 1/9 to 9/1.

The water-soluble polymer can be prepared by a common polymerization reaction. By regulating polymerization conditions, the molecular weight, molecular weight distribution, and polymerization composition of the water-soluble polymer to be obtained can be controlled. In order to increase polymer purity, the water-soluble polymer can be also purified by a method such as conventional dialysis, ultrafiltration, or solvent precipitation.

Preferable weight-average molecular weight (Mw) of the water-soluble polymer is from 2,000 to 500,000 and is more preferably from 5,000 to 100,000. Preferable molecular weight distribution is from 1.0 to 4.0, and is more preferably from 1.0 to 2.5 as Mw/Mn.

Moreover, preferable purity of the water-soluble polymer to be used in the reagent for latex aggregation reaction according to the present embodiment is 90% or more.

When the weight-average molecular weight, molecular distribution, or purity of the water-soluble polymer falls out of the above each range, there are cases where a sensitization effect on particle aggregation may be inhibited and the handling becomes difficult due to increased viscosity.

The water-soluble polymer may be a powder. In this case, the water-soluble polymer may be used after it is dissolved in an aqueous medium. Examples of the aqueous medium include distilled water and various buffers which may be usually used in the field of biochemistry, such as phosphate buffer, glycine buffer, Good's buffer, Tris buffer, and ammonia buffer. As the aqueous medium, phosphate buffer, glycine buffer, Good's buffer, and the like are more preferable.

### 1.2. Other components

For the reagent for latex aggregation reaction according to the present embodiment, the above water-soluble polymer may be used singly or may be used in combination with the other water-soluble polymer.

Examples of the other water-soluble polymer include polyethylene glycol, polyhydric alcohols, polyvinylpyrrolidone, carboxymethyl cellulose, methyl cellulose, ethyl cellulose, saccharose, dextran, pullulan, polyamino acids, alginic acid, and aminoethanesulfonic acid derivatives, aminopropanesulfonic acid derivatives, which are mentioned in the paragraph of Brief Decsription of the Background Art, as well as bovine serum albumin (BSA), casein, block ace, gum arabic, proteolytic products, amino acids, peptides, polypeptides, surfactants, and the like.

Furthermore, the reagent for latex aggregation reaction according to the present embodiment may contain additional components such as pH buffers, salts, and surfactants.

The concentration of the water-soluble polymer in the reagent for latex aggregation reaction according to the present embodiment is preferably from 0.01 to 30% by mass, more preferably from 0.1 to 5% by mass. When the concentration of the water-soluble polymer is less than 0.01% by mass, there is a case where a desired sensitization effect can not be obtained. On the other hand, when the concentration of the water-soluble polymer exceeds 30% by mass, there is a case where weighing error increases due to increased viscosity and non-specific reaction increases due to side reaction(s).

In the reagent for latex aggregation reaction according to the present embodiment, when the concentration of the water-soluble polymer falls within the range of from 0.01 to 30% by mass, the reagent is usually a colorless clear liquid. In this connection, at the measurement of the latex aggregation reaction, the amount of the reagent for latex aggregation reaction according to the present embodiment to be used may be adjusted according to each measuring item.

### 1.3. Directions for use

At the measurement of immunolatex aggregation, the concentration of the water-soluble polymer in a reaction system is preferably from 0.0001 to 10% by mass, more preferably from 0.0005 to 5% by mass. When the concentration of the water-soluble polymer in the reaction system is less than 0.0001% by mass, a sensitization effect may not be expressed in some cases.
On the other hand, when the concentration of the water-soluble polymer in the reaction system exceeds 10% by mass,
a sensitization effect becomes almost constant and rather, a side reaction inducing non-specific reaction becomes remarkable in some cases.

The reagent for latex aggregation reaction according to the present embodiment may be one prepared beforehand as a reagent or may be one prepared by adding the water-soluble polymer to the reaction system just before the start of immunoreaction.

The method for preparing the reagent for latex aggregation reaction according to the present embodiment may be suitably selected according to reagent constitution, required specification of actual diagnostic items, and a using situation at clinical site.

Furthermore, the reagent for latex aggregation reaction according to the present embodiment may be a reagent for both uses as an analyte diluent and an aggregation reagent, which contains component(s) for the analyte diluent and essential component(s) for the aggregation reagent in addition to the water-soluble polymer. The use of such a reagent can result in occurrence of proper aggregation reaction through only direct mixing with the reagent without previous dilution of an analyte.

### 2. Method for detecting target substance

The method for detecting a target substance according to one embodiment of the present invention includes a step of mixing the above water-soluble polymer; particles to which an antibody or antigen (referred to be the antibody or antigen-immobilized particles) for a predetermined target substance is immobilized; and an analyte, and a step of optically detecting aggregation of the particles formed in the mixing step.

### 2.1. Mixing step

In the above mixing step, although it is essential that the antibody or antigen-immobilized particles, the analyte, and the water-soluble polymer are co-present, the order of mixing them is not particularly limited.

Therefore, the antibody or antigen-immobilized particles, the analyte, and the water-soluble polymer may be simultaneously mixed or, before the step of mixing the above three components, any of the antibody or antigen-immobilized particles and the water-soluble polymer, the antibody or antigen-immobilized particles and the analyte, and the water-soluble polymer and the analyte may be mixed beforehand.

In the above mixing step, the mixing temperature is usually in the range of from 4 to 50°C. When the above temperature is lower than 4°C, there is a case where encounter of the antibody with the antigen decreases. On the other hand, when the above temperature exceeds 50°C, there is a case where stability of a complex of the antibody and the antigen decreases. The above temperature is preferably from 15 to 40°C, more preferably from 30 to 40°C.

In the above mixing step, the mixing time is usually 60 minutes or less. The pH of the mixed solution is usually in the range of from 5 to 10 and the pH of the mixed solution is more preferably from 6 to 9 in view of better stability of the complex of the antibody and the antigen.

In the method of detecting a target substance according to the present embodiment, in the above mixing step, the above water-soluble polymer is preferably mixed with the antibody or antigen-immobilized particles just before optical detection of the target substance. The following will describe the reason.

In general, sensitivity of the aggregation reaction of particles and stability of colloids have a relation of trade-off. Therefore, for example, when particle diameter of the particles is decreased to enhance the sensitivity of the aggregation reaction, the colloidal stability of the particles decreases. As a result, it becomes difficult to store the particles and thus storage stability of the reagent decreases. Therefore, in the method for detecting a target substance according to one embodiment of the present invention, by mixing the above water-soluble polymer with particles just before the detection of the target substance, the decrease in sensitivity of the aggregation reaction of the particles and colloidal stability can be reduced.

### 2.2. Step of optically detecting aggregation of particles

In the method of detecting a target substance according to the present embodiment, through the step of mixing the above water-soluble polymer, the antibody or antigen-immobilized particles, and an analyte which possibly contains the target substance, the above water-soluble polymer is brought into contact with the antibody or antigen-immobilized particles and the analyte.

In the method of detecting a target substance according to the present embodiment, in the step of optically detecting the aggregation of the above particles formed in the above mixing step, the aggregation reaction of the particles can be amplified to enhance sensitivity for detecting the target substance.

The "aggregation of particles" in the present invention means that repulsion among the antibody or antigen-immobilized particles is weakened by the reaction between the antigen and the antibody to bind the particles to one another.

The binding of the particles one another is multipoint type. Two antibody or antigen-immobilized particles bind at the initial stage, a plurality of the particles bind with the progress of the reaction, and finally, particle complexes composed of a large number of the particles is formed.

### 2.3. Analyte

In the method of detecting a target substance according to the present embodiment, an analyte (e.g., any of various biological liquid samples such as serum and plasma) is diluted to be appropriate concentration. In this case, by adding the water-soluble polymer to an analyte-diluting solution, the water-soluble polymer and the analyte can be mixed.

The analyte-diluting solution is usually an aqueous solution containing components such as a pH buffer, a protein including albumin or globulin, an amino acid, and/or a surfactant.

### 2.4. Antibody and antigen

Examples of the antigen include receptors, enzymes, blood proteins (e.g., carcinoembryonic proteins), infectious disease-related antigens (e.g., antigens of hepatitis B virus, hepatitis C virus, syphilitic pathogens, human immunodeficiency virus, pathogenic *Escherichia coli).* Examples of the antigen include antibodies against these antigens. The "antibody" herein also includes fragments of various antibodies and the like as long as they have binding ability to specific antigens.

### 2.5. Target substance

In the present invention, the "target substance" means a substance which may possibly cause immunoreaction and becomes a target to be detected, such as an antigen, a virus, a pathogen, an antibody, or an autoantibody.

### 2.6. Antibody or antigen-immobilized particles

The antibody or antigen-immobilized particles means water-insoluble carrier particles usually having an average particle diameter of from 0.1 to 10 µm to which an antibody or an antigen is immobilized. When the average particle diameter of the antibody or antigen-immobilized particles is less than 0.1 µm, there is a case where a sensitization effect on an optical signal by the particles is small. On the other hand, when the average particle diameter of the antibody or antigen-immobilized particles exceeds 10 µm, since the particle diameter is too large in comparison with the measuring wavelength, change in absorbance by aggregation decreases. Therefore, detection sensitivity may decrease in some cases.

Examples of the carrier particles to be used for the antibody or antigen-immobilized particles include red blood cells, carbon powders, bentonite, kaolin, micelles of lecithin, gelatin particles, synthetic latexes, and the like. Particularly, in view of particle diameter and strict regulation of particle diameter, latex particles whose main component is polystyrene are preferably used.

In the case where the latex particles are used as the carrier particles, if necessary, the surface may be chemically modified or a functional group may be introduced into the surface and then the surface may be chemically bound to an antibody or an antigen.

The method for preparing the carrier particles and the method for supporting an antigen or antibody for a target substance on the carrier particles are known to those skilled in the art.

### 2.7. Measuring apparatus

In the method for detecting a target substance according to the present embodiment, a known inspection machine can be used in the step of optically detecting aggregation of the particles. Examples of the apparatus capable of automatically tracing the particle aggregation include optical devices capable of detecting scattered light intensity, transmitted light intensity, absorbance, and the like.

As the method for optically measuring a degree of the aggregation of the particles, any known method can be used. Examples of the method include a turbidimetric assay method wherein the formation of aggregation is determined as an increase in turbidity, a method wherein the formation of aggregation is determined as a change in particle size distribution or average particle diameter, an integrating-sphere turbidity method wherein change in forward scattered light induced by the formation of aggregation is measured using an integrating sphere and the ratio to transmitted light intensity is compared, and the like. In the above measuring methods, a rate assay wherein at least two measured values are obtained at different time points and a degree of aggregation is determined based on an increase of the measured values between these time points, i.e., an increasing rate and an end point assay wherein one measured value is obtained at a certain time point which is usually considered to be an end point of the reaction and a degree of aggregation is determined based on the measured value can be utilized. A rate assay by turbidimetric assay method is preferable in view of convenience and rapidness of measurement.

Examples of an automatic machine for clinical laboratory tests suitable for the measurement of immunolatex aggregation reaction using the method for detecting a target substance according to the present embodiment include commercially available automatic analyzers such as Hitachi 7070, 7150, 7170, and LPIA-A700, S500.

According to the method for detecting a target substance according to the present embodiment, by including a step of mixing the above water-soluble polymer; antibody or antigen-immobilized particles; and an analyte, and a step of optically detecting aggregation of the above particles formed in the mixing step, the difference between absorbance change in a low value region and background is clear in the measurement of turbidity or absorbance. Thereby, sensitivity for detecting the target substance can be enhanced.

### 3. Examples

Although the following will further specifically describe the present invention with Examples, the present invention is not limited to thereto. In the present Examples, % and part(s) are on the basis of weight.

In the present Examples, weight-average molecular weight (Mw) and number-average molecular weight (Mn) were measured by gel permeation chromatography (GPC) using TSKgel α-M column manufactured by Tosoh Corporation under analyzing conditions of a flow rate of 1 mL/minute, an eluting solvent of an aqueous 0.1 mM sodium chloride solution/acrylonitrile mixed solvent, and a column temperature of 40°C, and using monodisperse polyethylene glycol as a standard.

### 3.1. Example 1 (Synthesis of Polyacrylamide)

In 900 g of water, 100 g of acrylamide and 2 g of cysteamine hydrochloride as a chain-transfer agent was mixed. The resulting mixture was placed into a separable flask fitted with a stirrer and was heated to 70°C under blowing of nitrogen therein. After addition of 1 g of 2,2'-azobis(2-methylpropionamidine) dihydrochloride as an initiator, polymerization was continued for 2 hours. After further heating to be 80°C for 3 hours as aging, cooling was carried out to be room temperature. The resulting aqueous solution was purified by dialysis to obtain an aqueous 6% polyacrylamide solution. The weight-average molecular weight (Mw) of the resulting polyacrylamide measured by GPC was 46,600 and Mw/Mn was 1.9.

### 3.2. Example 2 (Synthesis of Dimethylacrylamide-Methoxyethyl Acrylate Copolymer)

An aqueous 6% dimethylacrylamide-methoxyethyl acrylate copolymer solution was obtained in the same manner as in Example 1 except that 80 g of dimethylacrylamide and 20 g of methoxyethyl acrylate were used instead of 100 g of acrylamide and 4 g of cysteamine hydrochloride was used instead of 2 g of cysteamine hydrochloride. The weight-average molecular weight (Mw) of the copolymer measured by GPC was 23,600 and Mw/Mn was 2.4.

### 3.3. Example 3 (Immobilization of Antibody to Latex Particles)

Into a mixed solution of 10 mL of 1/15M phosphate buffer (pH 7.2) and 10 mL of physiological saline (hereinafter referred to as PBS), 1 mL of a 10% solution of latex particles for immunodiagnostics (product number: coat G0305 (average particle diameter: 0.31 µm, content of surface COOH group: 0.21 mmol/g-LTx, manufactured by JSR Co., Ltd.)) was dispersed to adjust the concentration of the particles to be 1% by mass.

Then, 1-ethyl-3-(3-dimethylaminopropyl)-carbodiimide hydrochloride (Dojindo Laboratories, hereinafter referred to WSC) was added thereto to adjust the final concentration to be 0.05%. An equivalent amount of 1 mg/mL solution of anti-CRP(C reactive protein) antibody (rabbit) was added to the particle dispersion was added, and the antibody (anti-CRP antibody) was immobilized to the surface of the particles under slow rotation at 56°C for 3 hours. Thereafter, 0.5 mL of 1% bovine serum albumin/0.05% Rapigest SF was added to the particle dispersion, followed by slow rotation with stirring at room temperature for 10 hours. After these operations, the particle dispersion was transferred into a centrifugation tube and subjected to centrifugation at 10,000 rpm for 20 minutes to collect the particles as a precipitate, and the supernatant containing unreacted antibody, WSC, and the like was removed. Subsequently, the particles were re-suspended in a 50mM Tris buffer of pH 7.4 and dispersed with ultrasonic wave for 10 minutes. The operation was repeated twice and finally the particles were dispersed with ultrasonic wave. After the resulting dispersion was passed through a 0.8 µm disc filter, the particles were adjusted using a solution of 10mM PBS buffer/0.02% bovine serum albumin/0.01% polyvinylpyrrolidone (molecular weight: 360,000) in order to adjust particle solid content to be 0.05%. A dispersion of antibody (anti-CRP antibody)-immobilized latex particles was obtained.

### 3.4. Example 4 (Preparation of Reagent for Latex Aggregation Reaction and Performance Evaluation)

Each of acrylamide (AAM) and dimethylacrylamide-methoxyethyl acrylate copolymer (DMAA/MEA) synthesized in the above Examples 1 and 2 were added as a sensitizer with water to a reagent under the conditions shown in Table 1. Moreover, using the reagent for latex aggregation reaction prepared, an immunolatex aggregation assay was carried out under the conditions shown in Table 1.

In the immunolatex aggregation measurement, a Hitachi 7020 model automatic analyzing apparatus was used and 3 µl of an analyte (a standard solution of a target substance (CRP antigen)), 150 µl of a first reagent (0.1% bovine serum albumin/PBS), 50 µl of a second reagent (a 0.05% dispersion of latex particles), and 50 µl of a third reagent (sensitizer) were used at a using wavelength of 570 nm and a measuring temperature of 37°C.

Moreover, in the present measurement, after mixing and stirring the analyte (target substance), the first reagent, the second reagent, and the third reagent, evaluation was performed by a rate assay method wherein the difference (change) between turbidity when 50 seconds had passed and turbidity when 200 seconds has passed was measured. Table 1 shows results of absorbance change in the case where the CPR antigen is at each concentration and the added amount of each sensitizer.

### 3.5. Example 5 (Evaluation of Measurement Stability)

In order to confirm measurement stability of the reagents for latex aggregation reaction using the polymers obtained in Examples 1 and 2, the reagents of test numbers 1, 2, 8, and 10 in the following Table 1 were repeatedly measured 20 times. CV (Coefficient of Variation) values measured in the case where the antigen concentration was 0.02 mg/dL were 8%, 6%, 10%, and 7%, respectively. Also, the CV values measured in the case where the antigen concentration was 10 mg/dL were 5%, 4%, 7%, and 5%, respectively.

From the above results, in the cases where the reagents for latex aggregation reaction using the polymers obtained in Examples 1 and 2 were used, it can be understood that the stability of the measured values are entirely not changed and rather slightly improved.

### 3.6. Example 6 (Evaluation of Stability with Time)

In order to confirm stability of the reagents for latex aggregation reaction using the polymers obtained in Examples 1 and 2, the reagents of test numbers 1, 3, 8, and 12 in the following Table 1 were stored at 4°C and 37°C for 1 year. The stored reagents at each temperature were measured at the same time 10 times. As a result, the measured values of the reagents from different storage conditions were all at the same level. These values were also the same as the values immediately after preparation and, when the measured values obtained for individual test numbers were statistically processed, the CV values at an antigen concentration of 0.1 mg/dL level were all 9% or less. As a result, since the values fall within the range of original measurement accuracy of the reagents of latex aggregation reaction using the polymers obtained in Examples 1 and 2 as a sensitizer, it can be understood that decrease in performance with lapse of time can be suppressed in the reagents of latex aggregation reaction using the polymers obtained in Examples 1 and 2.

**Table 1**

| Test No. | Kind of polymer | Target reagent for addition | Concentration of sensitizer (%) | Absorbance value at each antigen concentration | | | | | | |
|---|---|---|---|---|---|---|---|---|---|---|
| | | | | Antigen concentration (mg/dL) | | | | | | |
| | | | | 0 | 0.02 | 0.1 | 0.5 | 3 | 10 | 50 |
| 1 | AAM | First reagent | 0 | 48 | 45 | 100 | 240 | 366 | 550 | 574 |
| 2 | AAM | First reagent | 0.5 | 51 | 533 | 677 | 405 | 186 | 137 | 129 |
| 3 | AAM | First reagent | 1 | 97 | 619 | 433 | 321 | 111 | 103 | 105 |
| 4 | AAM | Second reagent | 0.1 | 44 | 85 | 256 | 398 | 466 | 659 | 674 |
| 5 | AAM | Second reagent | 1 | 46 | 238 | 377 | 506 | 683 | 650 | 666 |
| 6 | AAM | Third reagent | 0.01 | 44 | 78 | 142 | 291 | 421 | 587 | 594 |
| 7 | AAM | Analyte | 1 | 58 | 61 | 136 | 262 | 400 | 559 | 586 |
| 8 | DMAA/MEA | First reagent | 0 | 48 | 45 | 100 | 240 | 366 | 550 | 574 |
| 9 | DMAA/MEA | First reagent | 0.5 | 36 | 203 | 518 | 761 | 826 | 819 | 812 |
| 10 | DMAA/MEA | First reagent | 1 | 63 | 442 | 756 | 823 | 659 | 536 | 509 |
| 11 | DMAA/MEA | Second reagent | 0.1 | 44 | 106 | 398 | 485 | 605 | 689 | 708 |
| 12 | DMAA/MEA | Second reagent | 1 | 56 | 216 | 629 | 753 | 784 | 635 | 603 |
| 13 | DMAA/MEA | Third reagent | 0.01 | 50 | 68 | 134 | 276 | 419 | 560 | 694 |
| 14 | DMAA/MEA | Analyte | 3 | 49 | 85 | 196 | 321 | 598 | 659 | 705 |

This patent application is based on Japanese Patent Application No. 2007-193688 filed on July 25, 2007.

A reagent for latex aggregation reaction contains a water-soluble polymer having a repeating unit represented by the following formula (1): 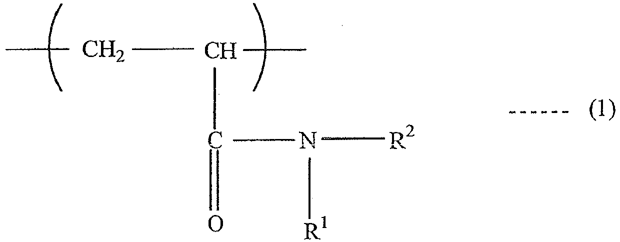 wherein R¹ and R² each independently represents a hydrogen atom or a substituted or unsubstituted alkyl group having 1 to 8 carbon atoms.

## Claims

1. A method for detecting a target substance comprising:
a step of mixing:
a water-soluble polymer, which provides a visually clear solution when the polymer is added to and mixed with pure water in order to adjust a solid content to be 1% at 25°C and having a repeating unit represented by the following formula (1): 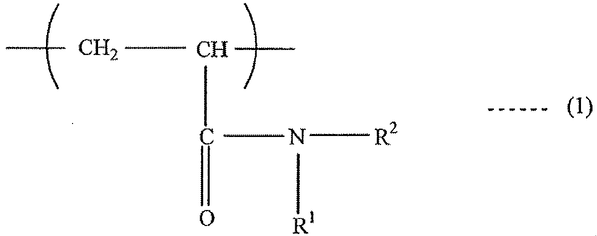
wherein R¹ and R² each independently represents a hydrogen atom or a substituted or unsubstituted alkyl group having 1 to 8 carbon atoms;
particles to which an antibody or antigen for a predetermined target substance is immobilized; and
an analyte which possibly contains the target substance,
a step of latex aggregation reaction in a mixed solution comprising the water-soluble polymer, the particles and the analyte, and
a step of optically detecting a degree of aggregation of the particles.

2. The method for detecting a target substance according to claim 1, wherein the water-soluble polymer further has a repeating unit represented by the following formula (2): 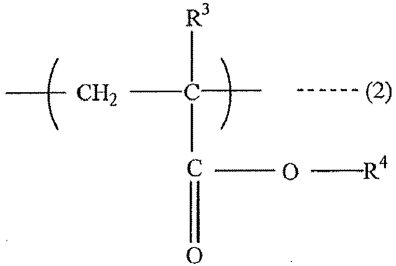 wherein R³ represents a hydrogen atom or a methyl group and R⁴ represents a substituted or unsubstituted alkyl group having 1 to 12 carbon atoms, an alicyclic hydrocarbon group, or an aromatic hydrocarbon group.

3. The method for detecting a target substance according to claim 1, wherein the above water-soluble polymer is at least one selected from polyacrylamide, polydimethylacrylamide, and a copolymer of an acrylamide monomer with a (meth)acrylate monomer.

4. Use of a water-soluble polymer, which provides a visually clear solution when the polymer is added to and mixed with pure water in order to adjust a solid content to be 1% at 25°C and having a repeating unit represented by the following formula (1): 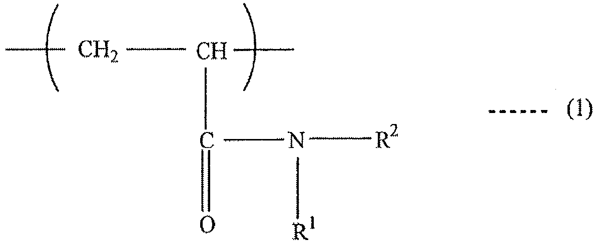 wherein R¹ and R² each independently represents a hydrogen atom or a substituted or unsubstituted alkyl group having 1 to 8 carbon atoms, as a reagent for a latex aggregation reaction in a method as defined in any one of claims 1 to 3.

## Patentansprüche

1. Verfahren für den Nachweis einer Zielsubstanz umfassend:
einen Schritt des Mischens:
eines wasserlöslichen Polymers, welches eine sichtbar klare Lösung bereitstellt, wenn das Polymer zu reinem Wasser gegeben und damit gemischt wird, um einen Feststoffgehalt von 1% bei 25°C einzustellen, und das eine durch die folgende Formel (1) dargestellte Wiederholungseinheit aufweist: 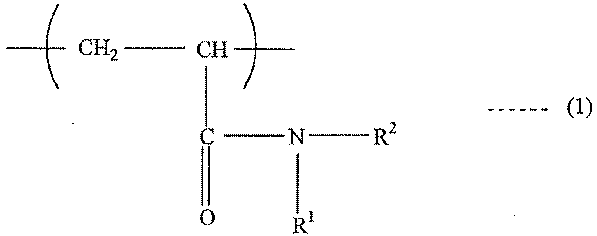
wobei R¹ und R² jeweils unabhängig ein Wasserstoffatom oder eine substituierte oder unsubstituierte Alkylgruppe mit 1 bis 8 Kohlenstoffatomen darstellt;
Teilchen, an welchen ein Antikörper oder ein Antigen für eine vorbestimmte Zielsubstanz immobilisiert ist; und
eines Analyts, welcher möglicherweise die Zielsubstanz enthält,
einen Schritt der Latexaggregationsreaktion in einer gemischten Lösung, welche das wasserlösliches Polymer, die Teilchen und den Analyt enthält, und
ein Schritt des optischen Nachweises eines Aggregationsgrades der Teilchen.

2. Verfahren für den Nachweis einer Zielsubstanz nach Anspruch 1, wobei das wasserlösliche Polymer außerdem eine durch die folgende Formel (2) dargestellte Wiederholungseinheit aufweist: 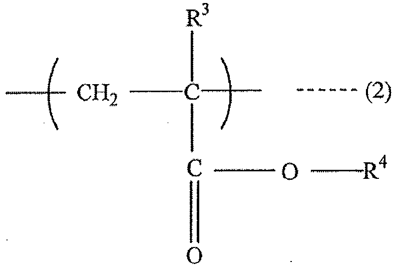 wobei R³ ein Wasserstoffatom oder eine Methylgruppe darstellt, und R⁴ eine substituierte oder unsubstituierte Alkylgruppe mit 1 bis 12 Kohlenstoffatomen, eine alizyklische Kohlenwasserstoffgruppe oder eine aromatische Wasserstoffgruppe darstellt.

3. Verfahren für den Nachweis einer Zielsubstanz nach Anspruch 1, wobei das vorstehende wasserlösliche Polymer wenigstens eines ausgewählt aus Polyacrylamid, Polydimethylacrylamid und einem Copolymer eines Acrylamidmonomers mit einem (Meth)acrylatmonomer ist.

4. Verwendung eines wasserlöslichen Polymers, welches eine sichtbar klare Lösung bereitstellt, wenn das Polymer zu reinem Wasser gegeben und damit gemischt wird, um einen Feststoffgehalt von 1% bei 25°C einzustellen, und das eine durch die folgende Formel (1) dargestellte Wiederholungseinheit aufweist: 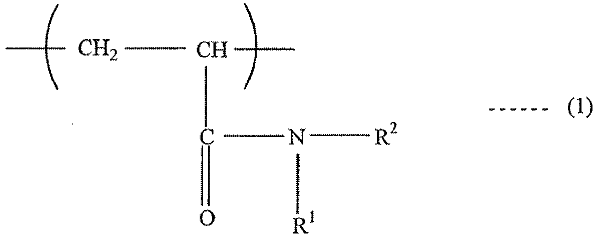 wobei R¹ und R² jeweils unabhängig ein Wasserstoffatom oder eine substituierte oder unsubstituierte Alkylgruppe mit 1 bis 8 Kohlenstoffatomen darstellt, als ein Reagens für eine Latexaggregationsreaktion in einem wie in einem der Ansprüche 1 bis 3 definierten Verfahren.

## Revendications

1. Procédé de détection d'une substance cible comprenant :
une étape de mélange :
d'un polymère soluble dans l'eau, qui fournit une solution visuellement limpide lorsque le polymère est ajouté et mélangé avec de l'eau pure afin d'ajuster une teneur en matières solides pour qu'elle soit de 1 % à 25 °C et ayant un motif de répétition représenté par la formule (1) suivante : 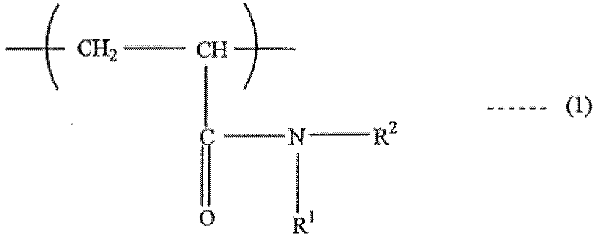
dans laquelle R¹ et R² représentent chacun indépendamment un atome d'hydrogène ou un groupe alkyle substitué ou non substitué ayant 1 à 8 atomes de carbone ;
de particules sur lesquelles un anticorps ou un antigène pour une substance cible prédéterminée est immobilisé ; et
d'un analyte, qui contient éventuellement la substance cible,
une étape de réaction d'agrégation de latex dans une solution mixte comprenant le polymère soluble dans l'eau, les particules et l'analyte, et
une étape de détection optique d'un degré d'agrégation des particules.

2. Procédé de détection d'une substance cible selon la revendication 1, dans lequel le polymère soluble dans l'eau comporte en outre un motif de répétition représenté par la formule (2) suivante : 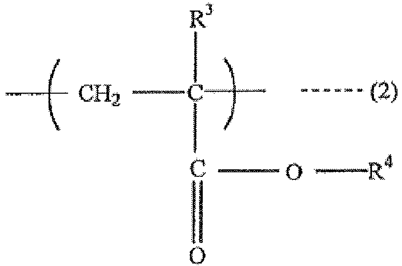 dans laquelle R³ représente un atome d'hydrogène ou un groupe méthyle et R⁴ représente un groupe alkyle substitué ou non substitué ayant 1 à 12 atomes de carbone, un groupe hydrocarboné alicyclique ou un groupe hydrocarboné aromatique.

3. Procédé de détection d'une substance cible selon la revendication 1, dans lequel le polymère soluble dans l'eau ci-dessus est au moins un polymère choisi parmi le polyacrylamide, le polydiméthylacrylamide, et un copolymère d'un monomère acrylamide avec un monomère (méth)acrylate.

4. Utilisation d'un polymère soluble dans l'eau, qui fournit une solution visuellement limpide lorsque le polymère est ajouté et mélangé avec de l'eau pure afin d'ajuster une teneur en matières solides pour qu'elle soit de 1 % à 25 °C et ayant un motif de répétition représenté par la formule (1) suivante : 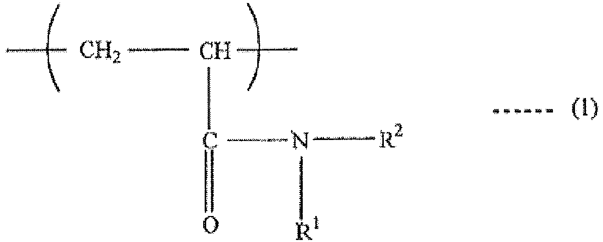 dans laquelle R¹ et R² représentent chacun indépendamment un atome d'hydrogène ou un groupe alkyle substitué ou non substitué ayant 1 à 8 atomes de carbone, en tant que réactif pour une réaction d'agrégation de latex dans un procédé tel que défini selon l'une quelconque des revendications 1 à 3.
